# EUROPEAN PATENT SPECIFICATION

(11) **EP 2 900 252 B1**
(45) Date of publication and mention of the grant of the patent: **27.12.2017**
(21) Application number: 13841655.7
(22) Date of filing: 27.09.2013
(51) Int. Cl.: A61K 9/00, A61K 38/06, A61P 37/04

(54) **AGENT FOR ENHANCING IMMUNITY CONTAINING GLUTATHIONE**
IMMUNITÄTSERHÖHUNGSMITTEL MIT GLUTATHION
AGENT D'AMÉLIORATION DE L'IMMUNITÉ CONTENANT DU GLUTATHION

(30) Priority: 28.09.2012 US 201261707286 P
(43) Date of publication of application: 05.08.2015
(73) Proprietor: Kyowa Hakko Bio Co., Ltd., Tokyo 100-8185 (JP)
(72) Inventor: OCHIAI, Masayuki, Tokyo 100-8185 (JP); MORITA, Masahiko, Tsukuba-shi Ibaraki 305-0841 (JP); MORISHITA, Koji, Tokyo 100-8185 (JP); KAGAMI, Erika, Tsukuba-shi Ibaraki 305-0841 (JP); RICHIE, John, Hershey, Pennsylvania 17033 (US)
(74) Representative: Vossius & Partner Patentanwälte Rechtsanwälte mbB
(86) International application number: PCT/JP2013/077018
(87) International publication number: WO 2014/051168

(56) References cited:
- EP-A1- 0 869 809
- WO-A1-2009/001884
- WO-A1-2009/099132
- DE-A1- 3 722 647
- DE-A1- 4 329 857
- JP-A- 2000 063 279
- US-A1- 2010 137 226
- DROEGE W ET AL: "GLUTATHIONE AUGMENTS THE ACTIVATION OF CYTOTOXIC T LYMPHOCYTE IN-VIVO", IMMUNOBIOLOGY, vol. 172, no. 1-2, 1986, pages 151-156, XP9188342, ISSN: 0171-2985
- JASON ALLEN ET AL: "Effects of Oral Glutathione Supplementation on Systemic Oxidative Stress Biomarkers in Human Volunteers", JOURNAL OF ALTERNATIVE AND COMPLEMENTARY MEDICINE, vol. 17, no. 9, 1 September 2011 (2011-09-01), pages 827-833, XP055246846, US ISSN: 1075-5535, DOI: 10.1089/acm.2010.0716
- RICHIE JOHN P ET AL: "Enhanced Glutathione Levels in Blood and Buccal Cells by Oral Glutathione Supplementation", FASEB JOURNAL, vol. 27, April 2013 (2013-04), page 862.32, XP002753803, & JOINT ANNUAL MEETING OF THE ASPET/BPS AT EXPERIMENTAL BIOLOGY (EB); BOSTON, MA, USA; APRIL 20 -24, 2013 ISSN: 0892-6638(print)

## Description

### Technical Filed

The present invention relates to a non-therapeutic oral use of glutathione or a salt thereof for enhancing immune function, wherein the agent is to be administered for a period of 3 months or more.

### Background Art

Reduced glutathione (GSH) is the most abundant thiol present in animal tissues (1-15 mM) (Non Patent Literature 1) and, together with its associated biosynthetic, redox and detoxification pathways represents the key defense system against oxidative stress and free radical damage in the cell (Non Patent Literatures 2-5). As the primary intracellular redox buffer, GSH is responsible for maintaining the thiol form of Cys in proteins, and protecting the cell against the constant assault of oxidative radicals. It also has many other critical functions including the detoxification of a variety of endogenous and exogenous compounds such as xenobiotics and carcinogens, preservation of protein structure and function, regulation of protein synthesis and degradation and modulation of immune function (Non Patent Literatures 6-8). Accordingly, preserving optimal blood and tissue levels of GSH is critical in maintaining health and the inability to synthesize GSH is lethal (Non Patent Literature 9). Numerous studies have demonstrated a wide variety of ill effects of even partial GSH depletion, including decreased immune function (Non Patent Literature 10), increased susceptibility to a wide range of xenobiotics (Non Patent Literature 11) and oxidative damage (Non Patent Literature 12). Changes in tissue and blood levels or production of GSH are symptomatic of many pathologies including infections (e.g. HIV), genetic diseases (e.g. Diabetes Mellitus), xenobiotic-induced diseases (e.g. alcoholic liver disease) and degenerative disorders (e.g. Parkinson's and Alzheimer's) and are closely associated with aging (Non Patent Literatures 13-15).

Despite the wealth of laboratory and epidemiological data demonstrating the efficacy of GSH administration, there remains little direct data regarding the effectiveness of oral GSH supplementation in human subjects.

### Citation List

### Non Patent Literature

[NPL 1] Meister, A., Glutathione metabolism and its selective modification. J Biol Chem, 1988. 263(33): p. 17205-8.
[NPL 2] Simone, G., M. Tamba, and M. Quintiliani, Role of glutathione inaffecting the radiosensitivity of molecular and cellular systems. Radiat Environ Biophys, 1983. 22(3): p. 215-23.
[NPL 3] Saez, G.T., Bannister, W.H., Bannister, J.V, Free radicals and thiol compounds - the role of glutathione against free radical toxicity. Glutathione: Metabolism and Physiological functions, ed. J. Vina1990, Boca Raton: CRC Press, Inc. 237-254.
[NPL 4] Sies, H., Oxidative Stress1985, New York: Academic Press.
[NPL 5] Sies, H., Glutathione and its role in cellular functions. Free Radic Biol Med, 1999. 27(9-10): p. 916-21.
[NPL 6] Meister, A. and M.E. Anderson, Glutathione. Annu Rev Biochem, 1983. 52: p. 711-60.
[NPL 7] vina, J., Glutathione: Metabolism and Physiological Functions. 1990, Boca Taton: CRC Press.
[NPL 8] Lu, S.C., Regulation of hepatic glutathione synthesis: current concepts and controversies. Faseb J, 1999. 13(10): p. 1169-83.
[NPL 9] Shi, Z.Z., et al., Glutathione synthesis is essential for mouse development but not for cell growth in culture. Proc Natl Acad Sci USA, 2000. 97(10): p. 5101-6.
[NPL 10] Robinson, M.K., et al., Glutathione depletion in rats impairs T-cell and macrophage immune function. Arch Surg, 1993. 128(1): p. 29-34; discussion 34-5.
[NPL 11] Jollow, D.J., Glutathione thresholds in reactive metabolite toxicity. Arch Toxicol Suppl, 1980. 3: p. 95-110.
[NPL 12] Ellouk-Achard, S., et al., Ex vivo and in vitro models in acetaminophen hepatotoxicity studies. Relationship between glutathione depletion, oxidative stress and disturbances in calcium homeostasis and energy metabolism. Arch Toxicol Suppl, 1995. 17: p. 209-14.
[NPL 13] Reid, M. and F. Jahoor, Glutathione in disease. Curr Opin Clin Nutr Metab Care, 2001. 4(1): p. 65-71.
[NPL 14] Macdonald, C.M., J. Dow, and M.R. Moore, A possible protective role for sulphydryl compounds in acute alcoholic liver injury. Biochem Pharmacol, 1977. 26(16): p. 1529-31.
[NPL 15] Richie, J.P., Jr., The role of glutathione in aging and cancer. Exp Gerontol, 1992. 27(5-6): p. 615-26.

### Summary of Invention

### Technical Problem

An objective of the present invention is to find out the effect of GSH supplementation based on biomarkers of immune function.

### Solution to Problem

The present specification discloses the following:
(1) A method for enhancing immune function, which comprises administering orally to a subject in need thereof effective amounts of glutathione or a salt thereof.
(2) A method for increasing natural killer cell cytotoxicity, which comprises administering orally to a subject in need thereof effective amounts of glutathione or a salt thereof.
(3) A method for increasing lymphocyte proliferation, which comprises administering orally to a subject in need thereof effective amounts of glutathione or a salt thereof.
(4) The method for enhancing immune function of (1), wherein the subject is a human and the administration is at a daily dose of 50 mg to 5000 mg in terms of the glutathione or a salt thereof.
(5) The method for enhancing immune function of (1), wherein the subject is a human and the administration is at a daily dose of 250 mg to 1000 mg in terms of the glutathione or a salt thereof.
(6) The method for enhancing immune function of (1), wherein the administration is for a period of 1 day to 1 year.
(7) The method for enhancing immune function of (1), wherein the administration is for a period of 1 week to 6 months.
(8) The method for enhancing immune function of (1), wherein the administration is for a period of 3 months.
(9) The method for enhancing immune function of (1), wherein the administration is for a period of 1 day or more.
(10) The method for enhancing immune function of (1), wherein the administration is for a period of 1 week or more.
(11) The method for enhancing immune function of (1), wherein the administration is for a period of 3 months or more.
(12) An oral agent comprising glutathione or a salt thereof for enhancing immune function.
(13) An oral agent comprising glutathione or a salt thereof for increasing natural killer cell cytotoxicity.
(14) An oral agent comprising glutathione or a salt thereof for increasing lymphocyte proliferation.
(15) Glutathione or a salt thereof for use in enhancing immune function.
(16) Glutathione or a salt thereof for use in increasing natural killer cell cytotoxicity.
(17) Glutathione or a salt thereof for use in increasing lymphocyte proliferation.
(18) A method for enhancing immune function, which comprises administering orally to a subject in need thereof effective amounts of glutathione or a salt thereof, provided that the method does not include any medical activity against a human.
(19) A method for increasing natural killer cell cytotoxicity, which comprises administering orally to a subject in need thereof effective amounts of glutathione or a salt thereof, provided that the method does not include any medical activity against a human.
(20) A method for increasing lymphocyte proliferation, which comprises administering orally to a subject in need thereof effective amounts of glutathione or a salt thereof, provided that the method does not include any medical activity against a human.
(21) Use of glutathione or a salt thereof in the manufacture of an oral agent for enhancing immune function.
(22) Use of glutathione or a salt thereof in the manufacture of an oral agent for increasing natural killer cell cytotoxicity.
(23) Use of glutathione or a salt thereof in the manufacture of an oral agent for increasing lymphocyte proliferation.

In particular, the present invention provides the following:
1. The non-therapeutic oral use of glutathione or a salt thereof for enhancing immune function wherein the agent is to be administered for a period of 3 months or more.
2. The use according to claim 1, wherein the glutathione is in form of food or drink.
3. The use according to claim 1, wherein the glutathione is in form of a food supplement.
4. The use according to claim 1, wherein the glutathione or a salt thereof is to be administered at a daily dose of 50 mg to 5000 mg.
5. The use according to claim 1, wherein the glutathione or a salt thereof is to be administered at a daily dose of 250 mg to 1000 mg.

### Advantageous Effects of Invention

According to the present disclosure, the immune function is enhanced and the natural killer cell (NK cell) cytotoxicity and lymphocyte proliferation are increased by glutathione or a salt thereof.

### Brief Description of the Drawings

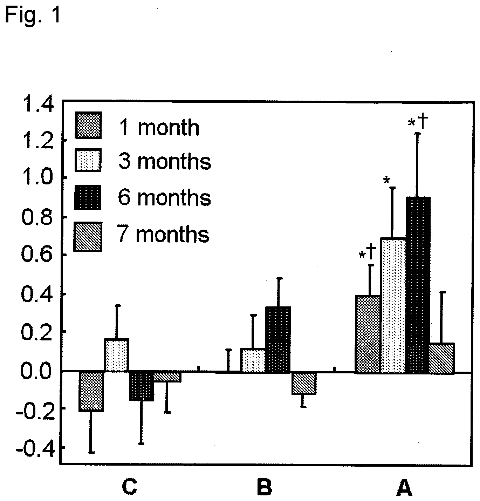

Fig. 1 shows changes in glutathione concentration in lymphocytes extracted from the subject after the GSH administration for 1, 3, 6, or 7 months, compared to glutathione concentration before the administration (0 month). The vertical axis indicates the glutathione concentration in lymphocytes (µmol/10⁶ cells). In the horizontal axis, C indicates placebo group, B indicates 250 mg/day of GSH administration group, and A indicates 1000 mg/day of GSH administration group. The bars in the same group of the horizontal axis respectively correspond to 1, 3, 6, and 7 months administration group from the left. Values are means ± standard error of the means. * indicates that the result of chi-square test between the baselines (before the GSH administration at each concentration) and the measured values of Group A after the GSH administration (1, 3, and 6 months) showed the significant difference (P<0.05). † indicates that the result of chi-square test between the placebo group (Group C) and the measured values of Group A after the GSH administration (1 and 6 months) showed significant difference (p<0.05)

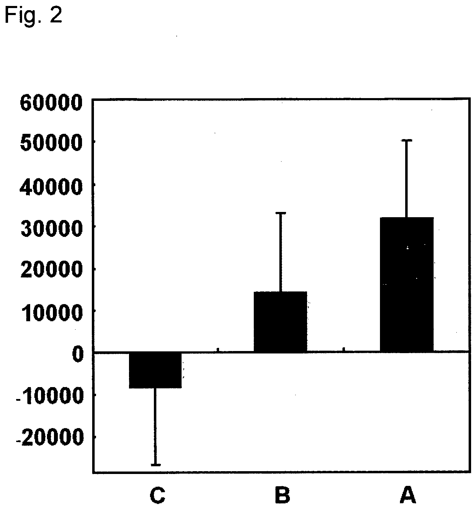

Fig. 2 shows changes in proliferation action of lymphocytes extracted from the subjects after the GSH administration for 3 months, compared to the proliferation action before the GSH administration (0 month). The vertical axis indicates the radioactivity due to the 3H-thymidine incorporated into the cell along with the cell proliferation (counts per minute (cpm)). In the horizontal axis, C indicates placebo group, B indicates 250 mg/day of GSH administration group, and A indicates 1000 mg/day of GSH administration group. Values are means ± standard error of the means.

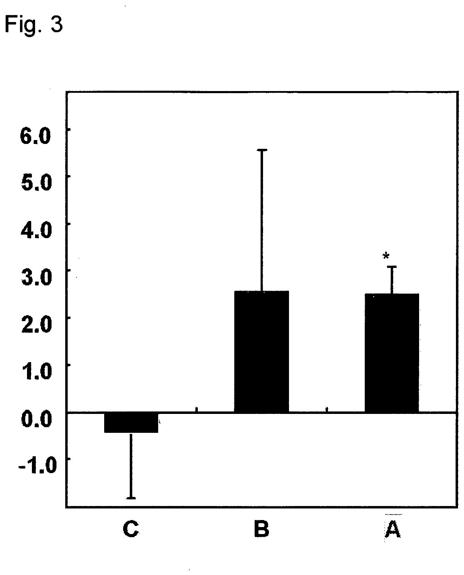

Fig. 3 shows changes in the cell cytotoxicity against human K562 cells of NK cells extracted from the subjects after the GSH administration for 3 months, compared to the cell cytotoxicity before the GSH administration (0 month). The vertical axis indicates ratio of radioactivity from K562 cells lysed by NK cell cytotoxicity to total radioactivity from K562 cells (% lysis). In the horizontal axis, C indicates placebo group, B indicates 250 mg/day of GSH administration group, and A indicates 1000 mg/day of GSH administration group. Values are means ± standard error of the means. * indicates that the result of chi-square test between the baselines (before 1000 mg of GSH administration) and the measured values of the above 1000 mg administration group showed significant difference (P<0.05).

### Description of Embodiments

Glutathione used in the present invention includes reduced glutathione (GSH) and oxidized glutathione. GSH refers to a tripeptide having the γ-L-Glu-L-Cys-Gly structure, and oxidized glutathione refers to a glutathione dipeptide in which two molecules of GSH are linked by an SS bond.

The GSH and the oxidized glutathione used in the present invention may be obtained by any production process. Examples of the processes for the production of GSH include the extraction method from microorganisms such as yeast (Methods in Enzymology, 3, 603 (1957)), the chemical synthesis method (Bull. Chem., Soc. Jpn., 53, 2529 (1980)) and the enzymatic method (Japanese Published Unexamined Patent Application No. 74595/86), and an example of the process for the production of oxidized glutathione is the method of Acta Biochim. Pol., 17, 175 (1970). GSH and oxidized glutathione can also be purchased from Sigma-Aldrich Corporation.

Examples of the salt of glutathione include hydrochloride, citrate, malate, α-ketoglutarate and aspartate, but the other salts or appropriate combinations of more than two salts may be used.

In the present invention, the term "immune function" refers to the function relating to the protection from microbial infection, the rejection of cells of other individuals, and the removal of mutant cells and wasted tissue. Examples of the immune function include the cell cytotoxicity by NK cells, lymphocyte proliferation, and the like.

"Natural killer cell (NK cell)" refers to the special lymphocyte which is not T-cell type and B-cell type and has ability of the protection from viral infection and the removal of tumor cells.

In the present invention, the term "enhancing immune function" means, for example, enhancement of the functions of the protection from microbial infection, the rejection of cells of other individuals, and the removal of mutant cells and wasted tissue by enhancing T cell proliferation and/or by enhancing the cell cytotoxic activity of NK cells against tumors and virus-infected cells.

The term "natural killer cell (NK cell) cytotoxicity" refers to the ability of NK cells to directly attack tumor cells, virus-infected cells, or the like to lead to their damage.

The term "increasing natural killer cell (NK cell) cytotoxicity" means enhancement of the action of NK cells to directly attack tumor cells, virus-infected cells, or the like to lead to their damage. Thus, increase in the NK cell cytotoxic action is useful as a method for treating and/or preventing tumors and viral diseases (Annual Review Immunity 2002, CHUGAI-IGAKUSHA, p. 76-80).

The term "lymphocyte proliferation" refers to the proliferation of T cells by cell division caused by antigen stimulation.

The term "increasing lymphocyte proliferation" means increase in the proliferation of T cells by cell division caused by antigen stimulation. This leads to increase in cytotoxic activity against intracellular and extracellular infectious microorganisms (Annual Review Immunity 2002, CHUGAI-IGAKUSHA, p. 10-11). As a result, the increase in lymphocyte proliferation is useful as a method for treating and/or preventing infectious diseases caused by the intracellular or extracellular infectious microorganisms.

Examples of the intracellular infectious microorganism include virus, chlamydia, rickettsia, listeria, leishmania, acidophil, Salmonella typhi, and the like. Examples of the extracellular infectious microorganism include protozoa, fungus, parasite, mycoplasma, enteritis coccus, Escherichia coli, and the like.

It has been reported that concanavalin A (Con A) induced lymphocyte proliferation activity and NK cell cytotoxic activity are increased in a GSH concentration dependent fashion in *in vitro* test using murine-derived splenic lymphocytes (Yakugaku Zasshi, 126(10), pp. 849-857, 2006). Further, the lymphocyte proliferation action by administering 2-melcaptoethanol (2-ME), which has a GSH production action *in vivo*, has been known (J. Immunol. 146(6), pp.1921-1927, 1991). Furthermore, it has been reported that glutathione concentration in immune cells, PHA stimulated lymphocyte proliferation, and NK cell cytotoxic activity are increased by oral administration of N-acetylcysteine (N-Ac), which is a precursor of glutathione, to a human.

In the present invention, glutathione may be administered as such, but it is usually preferred to be administered as various kinds of preparations. The preparations comprise glutathione or a salt thereof, as an active ingredient, and may further comprise any additional active ingredients. The preparations are produced according to any methods well known in the technical field of pharmaceutics by mixing the active ingredient with one or more kinds of pharmaceutically acceptable carriers.

It is desirable to administer the preparation by the route that is the most effective for enhancing immune function, namely administration.

The preparation is to be administered in the form of oral preparations (e.g., tablets, powders, granules, pills, suspensions, emulsions, infusions/decoctions, capsules, syrups, liquids, elixirs, extracts, tinctures and fluid extracts). Liquid preparations suitable for oral administration such as syrups can be prepared by adding water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), preservatives (e.g., paraoxybenzoic acid derivatives such as methyl paraoxybenzoate, and sodium benzoate), flavors (e.g., strawberry flavor and peppermint), and the like.

Tablets, powders, granules or the like suitable for oral administration can be prepared by adding excipients such as sugars (e.g., lactose, white sugar, glucose, sucrose, mannitol and sorbitol), starch (e.g., potato starch, wheat starch and corn starch), inorganic substances (e.g., calcium carbonate, calcium sulfate, sodium hydrogencarbonate and sodium chloride), crystalline cellulose and plant powders (e.g., licorice powder and gentian powder), disintegrating agents such as starch, agar, gelatin powder, crystalline cellulose, carmellose sodium, carmellose calcium, calcium carbonate, sodium hydrogencarbonate and sodium alginate, lubricants such as magnesium stearate, talc, hydrogenated vegetable oil, macrogol and silicone oil, binders such as polyvinyl alcohol, hydroxypropyl cellulose, methyl cellulose, ethyl cellulose, carmellose gelatin and starch paste, surfactants such as fatty acid esters, plasticizers such as glycerin, and the like.

The preparations suitable for oral administration may also comprise additives generally used in foods and drinks such as sweeteners, coloring agents, preservatives, thickening stabilizers, antioxidants, color developers, bleaching agents, fungicides, gum bases, bitter agents, enzymes, glazing agents, sour a gents, seasonings, emulsifiers, nutrient supplements, manufacture facilitating agents, flavors and spice extracts.

The preparations for oral administration may be used as foods and drinks such as a health food, a functional food, a food supplement and a food for specified health uses for enhancing immune function, as such or in any of the forms such as a powder food, a sheet-shaped food, a bottled food, a canned food, a retort pouched food, a capsule food, a tablet food, a liquid food and a health drink.

One or more kinds of auxiliary components selected from the above-described antiseptics, preservatives, excipients, disintegrating agents, lubricants, binders, surfactants, plasticizers, etc. for oral preparations can also be employed in these parenteral preparations.

The concentration of glutathione or a salt thereof for enhancing immune function of the present invention is appropriately determined according to the kind of the preparation, the effect expected by the administration of the preparation, etc. Glutathione or a salt thereof are usually contained in an amount of 0.1 to 100% by weight, preferably 1 to 100% by weight, particularly preferably 25 to 100% by weight.

When the glutathione or a salt thereof is administered according to the present invention, the dose and administration schedule vary depending on the administration route, the subject's age and body weight and the like. When the subject is a human, glutathione may be administered to at a daily dose of 50 mg to 5000 mg, preferably 250 mg to 1000 mg in terms of the glutathione or a salt thereof. The period of administration is of three months or more.

The agent according to the present invention can be used not only for humans but also for animals other than humans (hereinafter abbreviated as nonhuman animals).

Nonhuman animals include animals other than humans such as mammals, birds, reptiles, amphibians and fish.

In the case of administration to a nonhuman animal, the dose varies depending upon the age and kind of the animal, and the nature or degree of severeness of the symptom. Usually, the agent is administered once to several times per day in an amount to give a daily dose of 1 mg to 600 mg, preferably 2 mg to 200 mg, more preferably 4 mg to 60 mg per kg of body weight in terms of ornithine or a salt thereof and glutathione or a salt thereof.

There is no specific restriction as to the period of administration, but it is usually one day to one year, preferably one week to three months. In another embodiment, the period of administration may be one day or more, preferably one week or more, further preferably three months or more.

In addition, the present specification discloses an oral agent for enhancing immune function, an oral agent for increasing NK cell cytotoxicity, and an oral agent for increasing lymphocyte proliferation, which are prepared for administering glutathione or a salt thereof to a human at 50 to 5000 mg/day, preferably 250 to 1000 mg/day, for one day or more, preferably one week or more, more preferably 3 months or more.

### Example

The present invention is described below by Examples; however, the present invention is not limited to the following Examples.

### 1. Study Design and Methods

The design included the recruitment of healthy subjects (28-79 yr. of age, mean = 46.6 years) randomized into 3 groups (GSH at 1000 mg/day, GSH at 250 mg/day, and placebo). The target accrual of 48 subjects, 16 per group was based upon power calculations and results from a previous clinical trial with selenium (El-Bayoumy, K., et al., Cancer Epidemiol Biomarkers Prev, 2002. 11(11): p. 1459-65). Blood samples were obtained from all subjects at baseline. Subjects began supplementation according to the following schedule: Glutathione capsules were provided by the sponsor, Kyowa Hakko USA. Capsules were formulated, and subjects began supplementation according to the following schedule as follows:
- Group A, GSH: 500 mg/capsule and cellulose: 15 mg/capsule, (2 pills daily);
- Group B, GSH: 125 mg/capsule and cellulose: 360 mg/capsule, (2 pills daily);
- Group C (placebo), cellulose: 470 mg/capsule, (2 pills daily).

Eligible participants were randomly assigned to either placebo (Group C) or high (Group A) or low (Group B) dose glutathione groups. Supplementation continued for 6 months with biological samples collected at 1, 3 and 6 months after baseline. At 6 months, supplementation was discontinued. A final collection of biological samples occurred after a 1-month washout period.

Blood samples were collected from an antecubital vein into 3 tubes containing sodium heparin as an anticoagulant and immediately placed on ice. Blood samples were collected between 9:00 am and 1:00 pm and subjects were not fasted prior to collection. Tubes were mixed by gentle shaking and one 2.5 ml aliquot of whole blood was removed for analysis of neutrophil phagocytosis and respiratory burst (see below). Two 0.5 ml aliquots of whole blood were removed and stored at -80°C for future analyses. The remaining blood was centrifuged for 10 min at 1300 x g to obtain plasma, buffy coat and red cell fractions. Multiple 0.5 ml aliquots of plasma were placed into 1.5 ml cryovials and immediately frozen at -80°C. Packed red cells will be washed 3x in saline and aliquoted (0.5 ml each) into multiple cryovials and frozen at -80°C. Buffy coat fractions were combined and lymphocytes were isolated by Ficoll-Hypaque density gradient centrifugation. In brief, after addition of 3 ml of Ficoll, buffy coats were centrifuged at 400 g for 30 min at 19°C. Lymphocyte layers were removed and washed 2 times in PBS, followed by centrifugation at 250 g for 10 min. After the final wash, lymphocytes were resuspended in 5 ml PBS. Cell number was assessed after addition of 40 µl trypan blue to 10 µl of cell suspension using a hemocytometer. Cells were resuspended in 95% FBS, 5% DMSO at concentrations of 2.5 x 10⁶ cells/ml and stored in liquid nitrogen for analysis of lymphocyte proliferation or NK cell cytotoxicity (see below). Cells were kept on ice until acid extraction as described below.

*Glutathione* - Glutathione was measured in metaphosphoric (MPA) extracts of lymphocytes from each of the 4 time points (1, 3, 6, and 7 months). For lymphocytes, 400 µl of 5% MPA was added to aliquots of packed lymphocytes containing 5 x 10⁶ cells. After vigorous mixing and incubation at room temp for 15 min, samples were centrifuged at 14,000 g for 2 min. Supernatants and pellets were stored separately at -80°C until analysis of free and GSH, respectively. GSH was administered at 250 mg/day or 1000 mg/day and glutathione concentration in lymphocytes extracted from the subjects after 1, 3, 6, and 7 months from the start of administration was measured. The result is shown in Fig. 1.

*Lymphocyte Proliferation* - Lymphocyte samples from each of the 5 time points (0, 1, 3, 6, and 7 months) were thawed, washed 3 times and counted to determine cell viability. Cells were plated in triplicate and both 1x10⁵ and 5x10⁴ cells per cell in the presence and absence of the T cell mitogen phytohemagglutinin (PHA). Cell proliferation was monitored by 3H-thymidine incorporation. Intracellular radioactivity was measured by liquid scintillation counting and results were expressed as cpm. The evaluation results of lymphocyte proliferation action at the three-month time point (placebo, 250 mg/day administration group, and 1000 mg/day administration group) are shown in Fig. 2.

*NK Cell Cytotoxicity* - NK cell cytotoxicity was assessed using human K562 cells, labeled with sodium ⁵¹chromate as target cell. After labeling the K562 cells, they were placed in 96-well plates (1x10⁴ cells per well). Lymphocyte samples from each of the five time points were thawed, washed 3 times, counted to determine cell viability and added in triplicate to the wells at an effector:target cell ratio of 10:1. After incubation for 4 hr. at 37°C, cells were collected and supernatant analyzed for radioactivity by gamma counting. Results are expressed as percent of target cells lysed (% lysis) calculated as (cpm experimental-cpm spontaneous release)/(cpm maximum - spontaneous). The evaluation results of NK cell cytotoxic action at the three-month time point (placebo, 250 mg/day administration group, and 1000 mg/day administration group) are shown in Fig. 3.

This trial was designed to provide evidence of the efficacy of GSH and includes the assessment of both short-term (1-month) and long-term (6-month) effects. Based on previous laboratory animal studies and clinical data, it was anticipated that the effects of oral GSH supplementation would be progressive and cumulative. It was previously observed a similar ∼6 month progressive effect of selenium on blood GSH levels in healthy adult men (El-Bayoumy, K., et al., Cancer Epidemiol Biomarkers Prev, 2002. 11(11): p. 1459-65). In addition, the study design also allowed for the evaluation of effects of withdrawal of the supplement on the outcome variables. Doses were selected to optimize the likelihood that a biologically relevant response could be observed within the study time period, based upon previous clinical trial results.

### 2. Statistical Analysis of the Study

The primary evaluation of the effect of glutathione on key biomarkers (NK cell cytotoxicity, and lymphoproliferation) will involve comparison of the change from baselines to the end of the treatment, in each group using repeated measurement ANOVA. Alternatively, Student's t-tests and repeated measures ANOVA will be used to compare the mean change in biomarker levels over time in each age group.

### 3. Effects of oral glutathione on immune function markers in blood

The impact of GSH administration of several different blood-based parameters of immune function including lymphocyte proliferation, and NK cell cytotoxicity was examined. Lymphocyte and NK cell assays were performed on frozen purified lymphocyte cell fractions. These later assays are dependent on the yield of living and proliferative cells obtained from frozen cell preparations. Thus, all samples were carefully and quickly processed to obtain pure lymphocyte fractions which were then frozen down using a protocol designed to maintain optimal cell survival. Since, in these assays it is important to analyze all samples from each time point from a given individual all together on the same day to avoid assay-to-assay variation, it was necessary that viability be maintained in samples frozen for a minimum of at least 7 months. Cell samples that did not reach a minimum level of viability could not used for further analysis. Unfortunately, a large number of lymphocyte samples were lost to analysis for this reason.

This likely resulted from a -80°C freezer malfunction which occurred mid-study. While samples did not thaw, they did experience a period of warming temperatures of up to - 15°C. As a result of these lost samples, n-values for subjects which were usable pre- and post- samples was only 8-9 per group for the lymphocyte proliferation assay and 5-6 for the NK Cell cytotoxicity assay. Accordingly, statistical power, particularly for the subgroup analyses was negatively affected.

*Lymphocyte Total Glutathione* - The effects of oral GSH administration of lymphocyte levels are summarized in Fig. 1. Values are expressed on a per million cell basis and ranged from 0.7 to 6.7 µmol/10⁶ cells. A maximum increase of about 30% was observed after 6 months in the high dose GSH group. In the high dose group, the increase appeared to be time dependent with levels progressively increasing from 1 to 3 to 6 months of administration. Thus, at the high dose (1.0 g/day), GSH was accumulated in lymphocyte in an administration period-dependent fashion and there observed the significant difference in GSH concentration compared to that before administration (0 month). Therefore, a medical effect based on GSH (such as, enhancement of immune function) is expected to be expressed.

*Lymphocyte Proliferation -* The effects of GSH administration on lymphocyte proliferation are summarized in Fig. 2. Increases in mean proliferative capacity were observed after 3 months in a dose dependent fashion in both GSH groups. This suggested trends toward an increase in lymphocyte proliferation resulting from GSH administration. Therefore, GSH or a salt thereof is useful as an agent for enhancing immune function and, preferably, useful as an agent for enhancing the functions of the protection from microbial infection, the rejection of cells of other individuals, and the removal of mutant cells and wasted tissue by enhancing T cell proliferation and/or by enhancing the cytotoxic activity of NK cells against tumors and virus-infected cells. Further preferably, GSH or a salt thereof enhances the cytotoxic activity against intracellular infectious microorganisms (such as, virus, chlamydia, rickettsia, listeria, leishmania, acidophil, Salmonella typhi, or the like) and extracellular infectious microorganisms (such as, protozoa, fungus, parasite, mycoplasma, enteritis coccus, Escherichia coli, or the like) and is useful as an agent for treating and/or preventing infectious diseases caused by the intracellular or extracellular microorganisms.

*NK Cell Cytotoxicity -* The effects of GSH administration on NK cell cytotoxicity are summarized in Fig. 3. Increases in mean % lysis values were observed after 3 months in a dose dependent fashion in both GSH groups, but were only significant in the high GSH dose arm (Ppaired=0.01). Therefore, GSH or a salt thereof is useful as an agent for enhancing immune function and, preferably, useful as an agent for enhancing the functions of the protection from microbial infection, the rejection of cells of other individuals, and the removal of mutant cells and wasted tissue by enhancing T cell proliferation and/or by enhancing the cytotoxic activity of NK cells against tumors and virus-infected cells. Preferably, GSH or a salt thereof is useful as an agent for enhancing an action of NK cells to directly attack tumor cells and virus-infected cells to lead to their damage. Further preferably, GSH or a salt thereof is useful as an agent for treating and/or preventing tumors and viral diseases.

Overall, this was a highly successful trial which demonstrates for the first time the effectiveness of long-term GSH supplementation.

### Industrial Applicability

According to the present disclosure, the immune function is enhanced and the natural killer cell (NK cell) cytotoxicity and lymphocyte proliferation are increased by glutathione or a salt thereof.

## Claims

1. The non-therapeutic oral use of glutathione or a salt thereof for enhancing immune function wherein the agent is to be administered for a period of 3 months or more.

2. The use according to claim 1, wherein the glutathione is in form of food or drink.

3. The use according to claim 1, wherein the glutathione is in form of a food supplement.

4. The use according to claim 1, wherein the glutathione or a salt thereof is to be administered at a daily dose of 50 mg to 5000 mg.

5. The use according to claim 1, wherein the glutathione or a salt thereof is to be administered at a daily dose of 250 mg to 1000 mg.

## Patentansprüche

1. Nicht-therapeutische orale Verwendung von Glutathion oder einem Salz davon zur Förderung der Immunfunktion, wobei das Agens für einen Zeitraum von 3 Monaten oder mehr zu verabreichen ist.

2. Verwendung nach Anspruch 1, wobei das Glutathion in Form eines Nahrungsmittels oder eines Getränks vorliegt.

3. Verwendung nach Anspruch 1, wobei das Glutathion in Form eines Nahrungsergänzungsmittels vorliegt.

4. Verwendung nach Anspruch 1, wobei das Glutathion oder ein Salz davon in einer täglichen Dosis von 50 mg bis 5000 mg zu verabreichen ist.

5. Verwendung nach Anspruch 1, wobei das Glutathion oder ein Salz davon in einer täglichen Dosis von 250 mg bis 1000 mg zu verabreichen ist.

## Revendications

1. Utilisation orale non thérapeutique de glutathion ou d'un sel de celui-ci pour accroître la fonction immunitaire, dans laquelle l'agent doit être administré pendant une période de 3 mois ou plus.

2. Utilisation selon la revendication 1, dans laquelle le glutathion est sous la forme d'un aliment ou d'une boisson.

3. Utilisation selon la revendication 1, dans laquelle le glutathion est sous la forme d'un complément alimentaire.

4. Utilisation selon la revendication 1, dans laquelle le glutathion ou un sel de celui-ci doit être administré à une dose quotidienne de 50 mg à 5000 mg.

5. Utilisation selon la revendication 1, dans laquelle le glutathion ou un sel de celui-ci doit être administré à une dose quotidienne de 250 mg à 1000 mg.
